(19) Europäisches Patentamt
European Patent Office
Office européen des brevets

(11) Veröffentlichungsnummer: **0 065 069**
**B1**

(12) # EUROPÄISCHE PATENTSCHRIFT

(45) Veröffentlichungstag der Patentschrift: **30.04.86**

(51) Int. Cl.⁴: **C 08 F 291/00**, G 01 N 33/53, C 12 N 11/02, C 07 K 1/00

(21) Anmeldenummer: **82100784.6**

(22) Anmeldetag: **04.02.82**

(54) **Latex zur Immobilisierung von biologisch wirksamen Substanzen.**

(30) Priorität: **29.04.81 DE 3116995**

(43) Veröffentlichungstag der Anmeldung: **24.11.82 Patentblatt 82/47**

(45) Bekanntmachung des Hinweises auf die Patenterteilung: **30.04.86 Patentblatt 86/18**

(84) Benannte Vertragsstaaten: **AT BE CH FR GB IT LI NL SE**

(56) Entgegenhaltungen:
**GB - A - 2 005 275**
**GB - A - 2 041 940**

(73) Patentinhaber: **Röhm GmbH, Kirschenallee Postfach 4242, D-6100 Darmstadt 1 (DE)**

(72) Erfinder: **Krämer, Dieter, Dr., An der Favorite 4, D-6500 Mainz (DE)**
Erfinder: **Siol, Werner, Dr., Mühlbergstrasse 4, D-6102 Pfungstadt (DE)**
Erfinder: **Markert, Gerhard, Dr., Leipziger Strasse 25, D-6105 Ober-Ramstadt (DE)**
Erfinder: **Sütterlin, Norbert, Dr., Am Horeth 23, D-6105 Ober-Ramstadt (DE)**
Erfinder: **Fell, Cornelia, Rheinstrasse 36, D-6106 Erzhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 065 069 B1

LIBER, STOCKHOLM 1986

**Beschreibung**

Die Erfindung betrifft zur Immobilisierung (Fixierung) von biologisch wirksamen Substanzen geeignete Latices mit Kern-Schale-Aufbau, die für eine kovalente Fixierung geeignete funktionelle Gruppen im Schalenbereich aufweisen.

Das Problem der Trägerfixierung von biologisch wirksamen Substanzen stellt sich unter vielfältigen Aspekten im Bereich der Technik, beispielsweise in Biochemie und Biotechnologie, der Medizin, insbesondere der medizinischen Diagnostik u.a. In der Regel handelt es sich bei den zu fixierenden "biologisch wirksamen Substanzen" um Verbindungen bzw. Funktionseinheiten, die zur Wechselwirkung mit biologischen Systemen geeignet sind bzw. um diese biologischen Systeme selbst. Das besondere Interesse der Technik galt u.a. der Fixierung von Katalysatoren, speziell von Enzymen bzw. auch der Substratfixierung, wie sie z.B. für die Affinitätschromatographie von Bedeutung ist. Zur Erläuterung der bestehenden technischen Probleme wird im folgenden auf die Immobilisierung solcher "biologisch wirksamen Substanzen" näher eingegangen, die sich diagnostisch auswerten lassen.

Bei derartigen diagnostisch auswertbaren Reaktionen geht es um die Erfassung der Wechselwirkung von im Organismus vorhandenen oder vom Organismus produzierten Substanzen, die für den diagnostisch zu erfassenden Zustand symptomatisch sind, mit solchen Substanzen, die möglichst spezifisch auf diese "symptomatischen" Substanzen ansprechen. Ein außerordentlich hohes Maß an Spezifität kommt den Immunreaktionen zu. Immunreaktionen erfolgen bekanntlich zwischen Antigenen und Antikörpern: Einer der beiden Reaktionspartner muß bekannt sein, damit der andere qualitativ oder quantitativ in einer Korperflüssigkeit bestimmt oder in Zellen und Geweben lokalisiert werden kann.

Es gibt verschiedene analytische Verfahren zum Nachweis von Antigen-Anti-Körperreaktionen (Ag-AK-Reaktion), z.B. den Radioimmunoassay, den Enzymimmunoassay, die Immunofluoreszenz, die Immunodiffusion, insbesondere aber die Immunoagglutination

Die Immunoagglutination ermöglicht den Nachweis selbst niedriger Konzentrationen an immunologisch aktiven Materialien, indem man teilchenförmige Träger als Indikatoren benutzt, deren Verklumpung eine vorhandene Immunreaktion visuell oder photometrisch erfaßbar macht. Je nach Art der verwendeten Träger unterscheidet man zwischen Bakterien-, Harn-, Leukozyten-, Bentonit- und Latex-Agglutination. Verhältnismäßig große Aufmerksamkeit wurde der Latex-Agglutination geschenkt.

Die vorgeschlagenen Latices können verschiedenen Polymerisattypen angehören. Häufig Gebrauch gemacht wird von Latices auf Basis von Styrol bzw. styrolhaltigen Copolymeren (carboxyliertes Polystyrol, carbo xyliertes Polystyrol-Butadien-Copolymere, Styrol-Divinylbenzol, Styrol-Acrylamid, Acrylnitril-Butadien-Styrol, Styrol-Methacrylat) oder auf Basis von anionischen Phenolharzen, diazotierter Aminocellulose in Form feiner Partikel u.ä.

Auch Latices auf (Meth)acrylatbasis sind vorgeschlagen worden. Gemäß der US-PS 4 138 383 werden Polymerisate aus Acrylat-Mbnomeren, die OH, $-NH_2$ oder COOH-Gruppen enthalten, in Gegenwart von Vernetzern in Form von Suspensionen runder Mikrosphären mit einem gleichmäßigen Durchmesser $\leqslant$ 2000 Å hergestellt. An diese Latexmikrosphären werden Immunglobuline G (IgG) unter Verwendung von Carbodiimid oder Glutaraldehyd als Kondensationsmittel kovalent gebunden. Auch Versuche zur Modifikation des Latexaufbaus sind unternommen worden.

So werden in der DE-OS 28 40 768 Latices als Trager vorgeschlagen, an die eine wasserlösliche Polyhydroxyverbindung kovalent gebunden ist.

Die letztgenannte Anmeldung sieht eine Teilchengröße im Bereich von 0,01 bis etwa 0,9 µm und ein spezifisches Gewicht nahe dem des Wassers vor.

Das Latexmaterial soll im Hinblick auf immunologisch diagnostische Tests inert sein und aktive Gruppen aufweisen, die eine kovalente Bindung mit einer Polyhydroxyverbindung ermöglichen. Sofern diese aufgeführten Bedingungen erfüllt sind, sollen sich beliebige Latex-Polymere eignen.

In der BE-PS 874 588 werden Latexteilchen von 0,15-1,5 µ Durchmesser mit Schalenaufbau empfohlen. Dabei soll der Kem durch Polymerisation oder Copolymerisation "harter" Monomerer gebildet werden, die äußere Umhüllung durch Copolymerisation eines oder mehrerer "harter" Monomerer mit einer äthylenisch ungesättigten Verbindung, die freie Epoxygruppen aufweist.

Beispielhaft wird die radikalische Polymerisation von Styrol und Glycidylmethacrylat in Gegenwart eines Polystyrollatex beschrieben. Der so gebildete Latex kann z.B. mit Human-Chloriongonadotropin beladen werden.

Die technische Realisierung des Latex-Konzepts in der Immun-Diagnose ist aber bisher nicht über gewisse Schritte hinausgekommen.

Zu den limitierenden Faktoren gehören z.B. die folgenden: Bindung der biologisch wirksamen Substanzen (z.B. des Antikörpers).

Bislang werden die biologisch wirksamen Substanzen überwiegend adsorptiv an den Latex gebunden. Daraus entstehen fast zwangsläufig Probleme wegen der Diffusion der nur lose gebundenen Biomakromoleküle.

In einigen Fällen wird - wie bereits erwähnt - von einer kovalenten Bindung der biologisch wirksamen Substanzen Gebrauch gemacht. Im allgemeinen handelt es sich dabei um Bindungsfunktionen, deren Einführung in mehreren Verfahrensstufen vorgenommen werden muß, meist um die polymeranaloge Einführung von -COOH- oder $NH_2$-Gruppen sowie die anschließende Kupplung mit dem Protein mit Hilfe von (löslichen) Carbodiimiden oder Glutardialdehyd. Als Beispiel sei die mehrstufige kovalente Immobilisierung gemäß der DE-OS 28 12 845 genannt.

Aus der DE-OS 28 33 510 sind Latices mit Kern-Schale-Aufbau bekannt, bei denen der Kern ein Vinyl- und/oder Dienpolymerisat mit Carbonsäureund/oder Sulfonsäuregruppen und die Schale ein Vinylpolymerisat mit endständigen, aminsubstituierten Thiophenoläthergruppen darstellt. Die Aktivierung des Latex kann z.B. mittels Diazotierung erfolgen.

Anstelle der kovalenten Fixierung über einen Mehrstufenprozeß hat man auch versucht, Latices mit permanent reaktionsfähigen Gruppen, beispielsweise Oxirangruppen, zur Verfügung zu stellen. Diese weisen jedoch nur eine geringe Lagerstabilität auf. Als vielleicht gravierendster Nachteil der zur Fixierung biologisch wirksamer Systeme verwendeten Latices müssen die anschließend ebenso aufwendigen wie unerläßlichen Reinigungsoperationen betrachtet werden. Bei der kovalenten Fixierung von Proteinen an einem Latex müssen z.B. alle Hilfsstoffe (im Falle einer Carbodiimid-Kupplung die entstandenen Harnstoffe) und vor allem nicht gebundenes Protein in langwierigen Reinigungsschritten, beispielsweise durch Ultrafiltration entfernt werden. Diese zeitraubenden und aufwendigen Operationen schließen eine sinnvolle Verwendung hochwertiger, aber biologisch nicht besonders stabiler Materialien nahezu aus.

Die GB-A 2 041 940 betrifft gleichfalls Polymerlatices mit Kern-Schale-Aufbau, die zur Bindung biologisch wirksamer Substanzen dienen. Die Anwesenheit von vernetzenden Agentien in den Latices läßt sich dieser Druckschrift nicht entnehmen. Es gibt keinerlei Hinweis darauf, daß die Schale an sich hydrophil aufgebaut und nur infolge Vernetzung mit dem Kern verbunden bzw. verankert sein solle. Redispergierbarkeit ist für diesen Typ von Latices auszuschließen. Damit entfällt zum Beispiel der außerordentliche Vorteil auch beladene Latices redispergieren zu können.

Es bestand daher die Aufgabe, Latices zur Verfügung zu stellen, bei deren Anwendung die vorstehend geschilderten Nachteile nicht oder nur in geringem Umfang eintreten.

Der Technologie der Latices sind allerdings bestimmte, von den physikalischen Gegebenheiten herrührende Grenzen gesetzt:

Latex-Teilchen stellen bekanntlich an sich metastabile Systeme dar, die nur in Gegenwart von Tensiden und nur für einem begrenzten Zeitraum stabil gehalten werden können. Besonders gegenüber erhöhter Elektrolytkonzentration erweisen sich Latex-Teilchen als instabil. Da aber physiologisch relevante Vorgänge in elektrolythaltigen Lösungen (z.B. in 0,9 % Kochsalzlösung) ablaufen, ist die Handhabung der üblichen Latex-Teilchen sehr isr die Handhabung der ublichen Latex-Teilchen sehr schwierig, vor allem, wenn es sich um die für diagnostisches Arbeiten charakteristischen kleinen Substanzmengen handelt. Es können leicht Agglutinationen vorgetäuscht werden, sobald z.B. Antrocknungen auftreten oder auch nur Aufkonzentrierung zum Ausfallen der Latex-Teilchen führt. Eine Stabilisierung durch hohe Emulgatorkonzentration ist wegen deren denaturierender Wirkung auf biologische Systeme micht ratsam. Durch Einbau stark ionischer Gruppen kann zwar ein stabilisierender Effekt auf die Latex-Teilchen hervorgerufen werden, aber man beeinflußt damit gleichzeitig die für die biospezifische Wechselwirkung bestimmenden Eigenschaften der Latices nachhaltig.

Damit stellen sich eine Reihe von Forderungen an die zur Immobilisierung biologisch wirksamer Substanzen vorgesehenen Latices.

Die Latices sollen reaktive Gruppen ä fweisen, die eine kovalente Bindung der biologisch relevanten Moleküle unter physiologischen Bedingungen ermöglichen.

Die Latices sollen auch als wasserfreie Feststoffe gelagert werdem können, um eine Konstanz des Gehalts an reaktivem Gruppen über einem längeren Zeitraum zu gewährleisten.

Die Latices sollen völlig redispergierbar sein. Damit werden Antrocknungen bei der Handhabung unkritisch.

Die Latices sollen zentrifugierbar sein. Diese Bedingung ist dann erfüllt, wenn ihre Dichte von der Dichte des Trägermediums bzw. der kontinuierlichen Phase gemügend verschieden ist. Ist die Dichte der Teilchen höher als die des umgebendem Mediums, kann die Separierung der Teilchen mittels Sedimentation, ist sie geringer, kann sie durch Flotation vorgenommen werden.

Es wurde nun gefunden, daß sich zur kovalenten Immobilisierung von biologisch wirksamen Substanzen bzw. Strukturen, insbesondere im Hinblick auf eine diagnostische Anwendung, Polymerlatices mit Kern-Schale-Aufbau besonders eignen.

Gegenstand der Erfindung sind scmit Polymerlatices mit Kern-Schale-Aufbau zur Fixierung biologisch wirksamer Substanzen, dadurch gekennzeichnet, daß die Polymerlatices redispergierbar sind, wobei das Polymermaterial der Schale

I) zu 0,1 bis 20 Gew.-% aus einem radikalisch polymerisierbaren Vernetzer

II) zu 4,9 bis 99,9 Gew.-% aus radikalisch polymerisierbaren funktionellen Monomeren der Formel

$Z'-(R)_n-X$

wobei Z' für die radikalisch polymerisierbare Einheit

R für einen Abstandhalter

X für eine reaktive, nucleophil angreifbare Gruppe und

n für 0 oder 1 steht

zusammen mit radikalisch polymerisierbaren, hydrophilen Monomeren B mit der Maßgabe, daß der Anteil der funktionellen Monomeren am Gesamtpolymerisat der Schale wenigstens 0,1 Gew.-% beträgt, und

III) zu 0 bis 95 Gew.-% aus nicht oder nur begrenzt wasserlöslichen, radikalisch polymerisierbaren Monomeren A aus der Gruppe der Methacrylsäure- und Acrylsäureester sowie aus der Gruppe der Vinylester von Carbonsäuren aufgebaut ist, wobei Monomerenzusammensetzung der Schale so gewählt ist, daß

IV) der $T_{\lambda max}$-Wert der Schale im wasserfreien Zustand im Bereich von 20° C bis 250° C liegt,

und daß das Polymermaterial des Kerns durch Emulsionspolymerisation von radikalisch polymerisierbaren

Monomeren erzeugt worden ist.

Erfindungsgemäß besteht die Schale des Kern-Schale-Latexteilchens (K-S-Latex) aus einem mit Wasser quellbaren Material. Das Schalenmaterial soll von der Zusammensetzung her so hochgradig hydrophil sein, daß es ohne die Verankerung am Kernmaterial und/oder ohne Vernetzung zumindest teilweise in Wasser löslich wäre. Die Auflösung der Latex-Schale im umgebenden Wasser wird also durch die Bindung an den Latex-Kern, z.B. infolge Pfropfung und/oder Vernetzung verhindert.

Die Schale weist darüber hinaus die funktionellen Gruppen auf, die zur kovalenten Fixierung biologisch wirksamer Substanzen bzw. Strukturen notwendig sind. Vorzugsweise finden solche an sich bekannten funktionellen Gruppen Anwendung, die in wäßriger Lösung mit stärkeren Nucleophilen als Wasser reagieren und vom Wasser in dem physiologisch sinnvollen pH-Bereich, d.h. insbesondere im Bereich von 6,0 bis 9,0, insbesondere von 6,5 bis 8,0 nicht oder nur in untergeordnetem Maße angegriffen werden.

Die Auswahl der funktionellen Gruppen trägt der Tatsache Rechnung, daß das zu fixierende Material, insbesondere das Material biologischen Ursprungs als nucleophile Gruppe im allgemeinen die (freie) Aminogruppe, daneben gegebenenfalls noch Phenolische-, Hydroxy- oder Thiolgruppen aufweist.

Der Aufbau des Schalenanteils des erfindungsgemäßen Latex in seiner reaktiven Form kann daher in stark schematisierter Form wie folgt dargestellt werden:

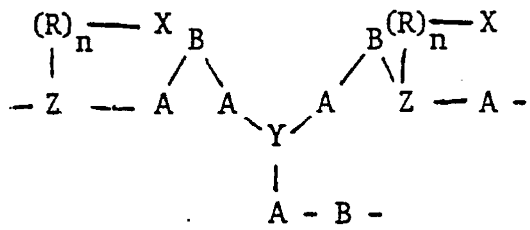

wobei X für die funktionellen Gruppen zur kovalenten Fixierung steht, vorzugsweise solche, die die vorstehend genannten Bedingungen erfüllen. R stellt dabei einen Abstandshalter (Spacer) zwischen funktionellen und polymerisierbaren Gruppen dar, Größe und Typ des Abstandshalters sind vergleichsweise unkritisch. In einer Reihe von Beispielen kann die Gruppe R ganz fehlen, d.h. n kann den Wert 0 oder 1 besitzen.

X hat in der Regel die Bedeutung einer von den in Frage kommenden Nucleophilen angreifbaren Gruppe, d.h. einer aktivierten Gruppe; vorzugsweise die Bedeutung einer Sulfonsäurehalogenid-, einer Thioisocyanatgruppe, eines aktivierten Esters, einer Thiocarbonyldioxy-, Carbonylimidoyldioxy-, Haloethoxy-, Haloacetoxy, Oxiran-, Aziridin-, Formyl-, Keto-, Acryloyl- oder Anhydridgruppe.

Als Sulfonsäurehalogenide kommen die Chloride und Bromide, als Haloacetoxy die Fluoro-, Chloro- und Bromoverbindungen, als Esterkomponente der aktivierten Ester, solche von HYdroxylaminverbindungen, wie des N-Hydroxysuccinimids oder des N-Hydroxyphthalimids, von (mittels elektronenanziehenden Gruppen) aktivierten Phenolen, wie von Halogenphenolen, wie Trichlorphenol oder von Nitrophenolen, von heterocyclischen Lactamen, wie Pyridon infrage.

Besonders bevorzugt sind Oxiran-, Keto-, Formyl-, Sulfonsäurechlorid-, Thioisocyanatgruppen sowie aktivierte Carbonsäureester sowie Carbonsäureanhydride. Bei den Monomeren des Typs $Z'-(R)_n-X$ stellt demnach Z' eine (radikalisch) polymerisationsfähige - Einheit und n 0 oder 1 dar.

Solche radikalisch polymerisatiomsfähige Einheiten sind z.B. Vinylgruppen, wobei Z' beispielsweise die Bedeutung

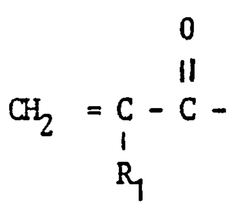

besitzt, worin $R_1$ für Wasserstoff oder Methyl steht bzw- für $CH_2\ COOR_2$,$CH_2$-$CONHR_2$ oder $CH_2$-$CON\ (R_2)_2$, wobei $R_2$ für einen Alkylrest mit 1 bis 4 Kohlenstoffatomen steht.

Desweiteren kann Z' sich von der Maleinsäure ableiten

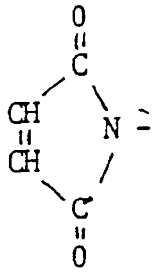

Als reaktionsfähige und zugleich polymerisierbare Einheiten sind ferner Maleinsäureanhydrid und Itaconsäureanhydrid anzusprechen sowie Acrolein, Methacrolein, Methylvinylketon und aktivierte Vinylester. Besonders bevorzugt sind Derivate der (Meth)acrylsäure umd des Maleiimids sowie Maleinsäure und Itaconsäureanhydrid.

Zur Verdeutlichung des Formelschemas Z'-R-X seien die folgenden Beispiele aufgeführt:

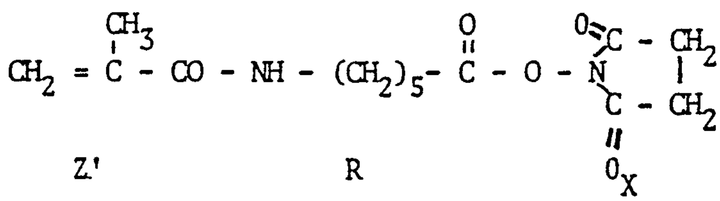

Z' R X

(Polymerisierbarer aktivierter Ester mit Spacer)

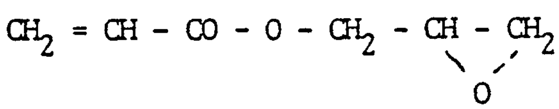 (Glycidylacrylat)

Z' R X

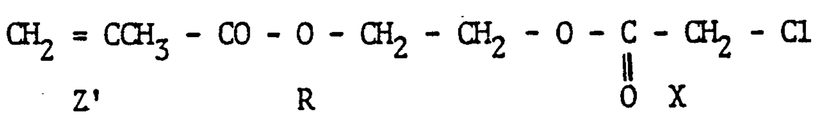

Z' R X

(2-(Chloroacetoxy)-äthylmethacrylat)

$CH_2 = CCH_3 - CO - O - C_6H_2Cl_3$

Z' X

(2,4,5-Trichlorphenylmethacrylat) R = 0

$CH_2 = C(CH_3) - COO - CH_2 - CH_2 - Br$

(2-Bromäthylmethacrylat)

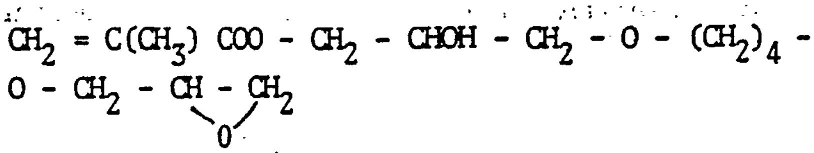

(Anlagerungsprodukt von Methacrylsäure an 1,4-Butandioldiglycidylether)

$CH_2 = CH - COO - CH_2 - CH_2 - O - CSNH - (CH_2)_6 - N = C = S$

(Anlagerungsprodukt von Acrylsäure-2-Hydroxyethylester an 1,6-Hexandiisothiocyanat)

$CH_2 = CH - O - CO - CH_2 - Cl$ (Chloressigsäurevinylester)

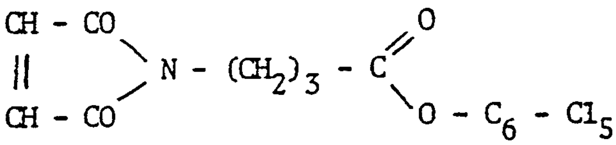

(4-Maleimido-buttersäure-pentachlorphenylester)

$CH_2 = C(CH_3) - COO - C_6H_4 - SO_2 - CH_3$

((4-Methylsulfinylphenyl)-methacrylat)

$CH_2 = CH - COO - CH_2 - C \equiv C - H$ (Propargylacrylat)

Bei den übrigen, am Aufbau der Schale beteiligten Einheiten (A und B in der schematischen Darstellung) handelt es sich definitionsgemäß um solche, die der Schale die zu fordernden Eigenschaften, nämlich die Hydrophilie und die Härte verleihen. Als Anhalt für die erwünschte Härte im wasserfreien Zustand gilt

$T_{\lambda max}$: 20 - 250°C, insbesondere 50 - 200°C (nach DIN 53445).

Andererseits sollten die am Aufbau der Schale beteiligten Monomeren selbst zweckmäßigerweise keine stark nucleophilen Gruppen (wie z.B. $-NH_2$, -SH) enthalten. Weiter sollte die Schale vorzugsweise keinerlei aromatische Reste aufweisen. Weiter müssen die Bestandteile der Schale in irgendeiner Weise in sich vernetzt sein. Für diese Vernetzung bzw. für die Verknüpfung mit dem Kern steht Y als Symbol.

Im Sinne der schematischen Darstellung seien weiter die in erster Linie für die Hydrophilie des Schalenanteils verantwortlichen Bestandteile als B, weitere Bestandteile, deren Auswahl in erster Linie auf die resultierende Härte des Gesamtpolymerisats abgestimmt werden muß, als A bezeichnet.

Die für den Schalenaufbau des erfindungsgemäß zu verwendenden Latex genannten Bedingungen werden z.B. durch Copolymerisate von Methacrylat- und/oder Acrylattyp erfüllt, wobei der qualitative und der quantitative Anteil so zu bemessen ist, daß die vorstehend angegebenen Kriterien für die Schale des Polymerlatex erfüllt sind.

Als hydrophile Bestandteile B kommen z.B. gegebenenfalls substituierte Methacrylamide und Acrylamide der allgemeinen Formel I

$$CH_2 = \underset{\substack{|\\ R_1}}{C} - CONR_3R_4 \qquad I$$

worin $R_1$ Wasserstoff oder Methyl und $R_3$ und $R_4$ unabhängig voneinander Wasserstoff cder einen Alkylrest mit 1 bis 4 Kohlenstoffatomen bedeuten, also unsubstituierte Amide sowie die mit primären und sekundären Aminen gebildeten Amide infrage. Besonders genannt seien (Meth)acrylsäureamid, N-Methyl(bzw. Isopropyl- oder -Butyl)-(meth)-acrylsäureamid, N,N-Dimethyl-(meth)acrylsäureamid, desweiteren (Meth)acrylsäuremorpholid (Sonderfall, in dem der Stickstoff über $R_4$ und $R_3$ Teil eines Rings ist) und N-Vinylpyrrolidon-2.

Ferner gehören hydroxygruppenhaltige Mcncmere des Acrylat- oder des Methacrylattyps, insbesondere hydroxygruppenhaltige Ester oder Amide der Acryl- und der Methacrylsäure sowie Alkoxyalkylester- und/oder Amide der Acryl- und der Methacrylsäure zu den hydrophilen Monomeren des Typs B, z.B. Vertreter der allgemeinen Formel II

$$CH_2 = \underset{\substack{|\\ R'_1}}{C} - CO - [Q - (CH_2)_p]_m - OR'_2 \qquad II$$

worin $R_1$ Wasserstoff cder Methyl, $R'_2$ Wasserstoff oder eine Alkylgruppe mit 1 bis 4 Kohlenstoffatomen, Q Sauerstoff oder eine Gruppe $-NR_2''$, worin R'' für Wasserstoff oder eine Alkylgruppe mit 1 bis 4 Kohlenstoffatomen steht, p eine ganze Zahl von 1 bis 3, vorzugsweise 2, und m eine ganze Zahl von 1 bis 25 bedeutet, mit der Maßgabe, daß, wenn Q für Sauerstoff steht, p nicht gleich 1 sein soll. Besonders genannt sei das Hydroxyäthylacrylat, Hydroxyäthylmethacrylat, 2-Hydroxyäthyl(meth)acrylamid, 2-Hydroxypropyl(meth)acrylamid, Monoester der (Meth)acrylsäure des Glycerins und anderer Polyole.

Weiter fallen unter den Monomerentyp B Sulfoäthylacrylate und Sulfoäthylmethacrylate sowie Sulfoäthylacrylamide und Sulfoäthylmethacrylamide. Ebenfalls als hydrophile Gruppen in die Schale des Latex eingebaut werden können polymerisierbare Säuren, wie (Meth)acrylsäure, Itaconsäure oder Maleinsäure oder polymerisierbare tert. Amine, wie 2-N,N-Dimethylaminoäthyl-(meth)acrylamid bzw. -(meth)acrylsäureester oder

3-N,N-Dimethylaminopropyl-(meth)acryl-amid bzw. -ester. Zur Vermeidung einer einseitigen Aufladung der Latexteilchen sollten diese sauren bzw. basischen Gruppen stets gleichzeitig in einem Teilchen vorhanden sein (z.B. Methacrylsäure und 2-N,N-Dimethylaminoäthyl-(methacrylat).

Als Monomere des Typs A kommen nicht oder höchstens begrenzt wasserlösliche Monomere in Frage, wobei der qualitative und der quantitative Anteil so zu bemessen ist, daß das vorstehend aufgeführte Kriterium der Härte des resultierenden Polymerisats erfüllt wird.

a) Ester der Acryl: und/oder der Methacrylsäure, mit $C_1$-$C_{20}$-Alkoholen, insbesondere der Methyl-, Äthyl- sowie die Propyl- und Butylester der Methacrylsäure, sowie der Methyl-, Äthyl-die Propyl- und Butylester und 2-Äthylhexylester der Acrylsäure,

b) copolymerisierbare Monomeren vom Typ des Vinylacetats, insbesondere Vinylacetat, Vinylpropionat, Vinylbutyrat und Vinylisobutyrat.

Es versteht sich, daß die sogenannten "weichen" Monomeren des Typs A nur in untergeordneter Menge vorhanden sein können, im allgemeinen weniger als 50 Gew.-%, bezogen auf das Polymere der Schale.

Die Härte bzw. die sonstigen relevanten Eigenschaften der Polymerisatfilme aus den individuellen Monomeren ist bekannt, desgleichen ihr Beitrag zu den Eigenschaften von Copolymerisaten [vgl. US-PS 2 795 564 H. Rauch-Puntigam, T. Völker in "Acryl- und Methacrylverbindungen", Springer-Verlag, Berlin 1967, S. 303-304, T.G. Fox, Bull. Am. Phys. Soc. 1, 123 (1956)].

Der Anteil des Vernetzers Y ist so zu bemessen, daß ein Abschwimmen der Latex-Schale nicht mehr möglich ist, in der Regel ist dazu wenigstens 0,1 Gew.-% erforderlich. Größere Mengen Vernetzer wirken sich keineswegs störend aus, so daß in der Regel Anteile von 0,1 bis 20 %, insbesondere 1 bis 10 Gew.-%, verwendet werden.

Unter chemischen Gesichtspunkten kann Y jedes multifunktionelle Acrylat cder Methacrylat sein, z.B. Glykoldimethacrylat, Butandioldiacrylat, Triäthylenglykoldimethacrylat, Tetraäthylenglykoldiacrylat, Pentaerythrittetraacrylat u.a. mehr. Dabei müssen nicht alle funktionellen OH-Gruppen des dem Vernetzungsmittel zugrundeliegenden Polyols mit polymerisierbaren Säuren verestert sein (z.B. hat Pentaerythritdimethacrylat 2 freie OH-Gruppen), so daß diese Vernetzungsmittel auch durchaus hydrophilen Charakter aufweisen können. Ein anderes Beispiel für ein hydrophiles Vernetzungsmittel Y ist das N,N-Methylenbis(methacrylamid).

Daneben sind natürlich auch solche Monomere als Vernetzer einsetzbar, die neben einer gut polymerisierbaren Gruppe leicht pfropfbare Einheiten enthalten, z.B. Allylmethacrylat.

Der Kern der erfindungsgemäßen Kern-Schale-Latices ist nicht eigentlich kritisch, sofern die Bedingung erfüllt ist, daß die resultierenden Latexteilchen formstabil sind, d.h. in sich genügende Starrheit aufweisen. Im Hinblick auf die Forderung der Technik soll die Redispergierbarkeit des Kern-Schale-Latexsystems gewährleistet sein. Das Polymermaterial des Kerns kann diese Forderungen beispielsweise sowohl dadurch erfüllen, daß es sich um ein an sich weiches aber stark vernetztes Polymerisat handelt als auch dadurch, daß es sich um ein an sich hartes (vernetztes oder nicht vernetztes) Polymerisat handelt. Es liegt in der Natur des Kern-Schale-Aufbaus, daß störende Interaktionen, die vom Kern-Material ausgehen können, weniger zu befürchten sind, so daß auch unter diesem Aspekt eine relative Freiheit der Wahl besteht. Es kann daher der Kern Träger der zur einer physikalischen Trennung bzw. Identifikation geeigneten Eigenschaft, z.B. Träger einer auf physikalischem Wege feststellbaren Markierung sein. Dabei ist z.B. an Farbstoffe, Fluoreszenzfarbstoffe u.ä. zu denken. Weiter kann der Kern infolge seines vom umgebenden Medium abweichenden Gewichts die physikalische Separierung ermöglichen.

Es ist demnach ein Aufbau des Kernmaterials aus solchen Monomeren bzw. Camonomeren möglich, die mit der Forderung der Redispergierbarkeit des Latex kompatibel sind, z.B. alle Copolymerisatzusammensetzungen aus Derivaten der Methacrylsäure und der Acrylsäure sowie verschiedener Vinylester, die dem Copolymerisat eine $T_{\lambda max}$ (nach DIN 53445) von wenigstens 0° C verleihen; z.B. den Copolymerisaten aus Methylmethacrylat, Butylmethacrylat, Methylacrylat u.v.a. mehr. Während im Bereich des Schalenmaterials stets auf das Nichtvorhändensein von aromatischen Gruppierungen zu achten ist (potentielle Hapteneigenschaften) können im Kern des Latex natürlich auch Monomere vom Typ des Styrols verwendet werden, z.B. Styrol, Vinyltoluol, Divinylbenzol und damit auch Copolymerisate aus Styrol und Maleinsäurebzw. Fumarsäureester.

Liegt die Glastemperatur des Kernpolymerisats deutlich $< 0°C$, so empfiehlt sich die Mitverwendung von wenigstens 1 % Vernetzer, z.B. Glykoldimethacrylat, Divinylbenzol etc. Im Zusammenhang mit der Forderung nach einer von der Dichte des Trägermediums bzw. der kontinuierlichen Phase abweichenden Dichte des Gesamtsystems, kommt vor allem solchen Monomeren, die dem Latex eine erhöhte Dichte verleihen, besondere Bedeutung zu.

Es bieten sich insbesondere "schwere" Monomere, also speziell solche mit einem oder mehreren Halogenatomen, speziell chlorierte oder bromierte Monomere an. Genannt seien beispielsweise Vinylverbindungen, wie Vinylchlorid, Styrolderivate, wie Chlor- oder Bromstyrol sowie Derivate der (Meth)acrylsäure, die diese schweren Gruppen in der Seitenkette tragen, z.B. das 2,4,6-Tribromphenoxyäthylmethacrylat.

Alternativ kann man auch den Kern aus Monomeren aufbauen, deren Dichte als Polymere sich von der Dichte des Trägermediums bzw. der kontinuierlichen Phase weniger stark unterscheidet. In einem solchen Falle ist die Größe des Kerns soweit zu steigern, daß dennoch eine gute Separierbarkeit gewährleistet ist.

Die Herstellung der Kern-Schale-Latices der erfindungsgemäß anwendbaren Art kann in Anlehnung an die an sich bekannten Verfahren erfolgen. (Vgl. DE-AS 27 22 752). Beispielhaft für bevorzugte Ausführungsformen seien

im folgenden die Verfahren zur Herstellung eines grobteiligen bzw. feinteiligen Kern-Schale-Latexmaterials angegeben.

Ob ein Latex grobteilig oder feinteilig werden soll, wird zweckmäßigerweise an Kernmaterial festgelegt. Die Herstellung eines grobteiligen Polymerisatkerns kann beispielsweise durch eine völlig emulgatorfreie Polymerisation erfolgen.

Eine vorteilhafte Ausführungsform besteht darin, das Monomer oder die Monomermischung innerhalb 0,5 bis 4 Stunden in eine auf ca. 50 bis 100°C aufgeheizte Wasservorlage tropfen zu lassen, die eine ausreichende Menge eines wasserlöslichen Initiators, wie beispielsweise Kalium- oder Ammoniumperoxidisulfat, Wasserstoffperoxid oder Salze der 4,4'-Azobis(cyanovaleriansäure) enthält. Statt einer thermischen Polymerisation im Bereich von ca. 50 bis 100°C kann jedoch die Reaktion mit Hilfe eines Redox-Initiatorsystems auch bei niedrigerer Temperatur initiiert werden. Auch öllösliche Starter, wie beispielsweise Dibenzoylperoxid oder Azoisobuttersäuredinitril sind als Polymerisationsinitiatoren geeignet. In diesem Falle ist die Mitverwendung von wenigstens geringen Mengen Emulgator vorteilhaft oder sogar notwendig. Ein anderer Weg zur Erzielung großer Latexteilchen führt über ein mehrstufiges Verfahren unter Zuhilfenahme von Saatlatex. In diesem Fall polymerisiert man in einer zweiten oder noch einigen nachfolgenden Stufen das gewünschte Monomer oder die Monomermischung auf einen vorher beliebig zubereiteten Saatlatex auf. Als Verfahrensweise kommen sowohl Batch-, Mehrfach-Batch- als auch besser Monomeren- bzw. Emulsionszulauf in Frage. Wesentlich für diese Ausführungsform ist, daß die Gesamtemulgatorkonzentration in den auf den Saatlatex folgenden Stufen so niedrig gehalten wird, daß alles Monomer auf diese Saatlatex-Teilchen aufpolymerisiert und keine Teilchenneubildung erfolgt. Besonders große Polymerisatkerne werden dann erhalten, wenn man als Saatlatex die zuerst beschriebenen, ohne Emulgator hergestellten grobteiligen Latices verwendet (vgl. EP-OS 79101398.0 bzw. DE-OS 28 33 601).

Grobteilige Systeme erhält man auch, wenn man in einer ersten Stufe einen Saatlatex herstellt, der ein Polymerisat sehr niedrigen Molekulargewichts enthält. Diese Latexteilchen können mit Monomer oder Monomergemischen angequollen und zu großen Latexpartikeln polymerisiert werden. Die Mitverwendung untergeordneter Mengen einer völlig wasserunlöslichen Substanz mit dem Monomer oder Monomergemisch kann ähnliche Wirkung wie das niedermolekulare Polymer haben (vgl. DE-OS 27 51 867, EP-P 0 003 905). Grobteilige Kerne z.B. von ca. 0,5 bis > 2 µm Durchmesser sind vorteilhaft, wenn die Dichte des Kern-Polymerisats nicht stark von der Dichte des Trägermediums bzw. der kontinuierlichen Phase abweicht.

Die Herstellung eines feinerteiligen Polymerisatkerns stellt im Prinzip die Synthese eines Latex nach denbekannten Kriterien der Emulsionspolymerisation dar, wobei die gewünschte Größe der Kern-Latex-Teilchen durch die Emulgatorkonzentration zu Beginn der Polymerisation eingestellt wird. Aufgrund der Tatsache, daß die Zusammensetzung des Kernmaterials relativ umkritisch ist, ist es im Prinzip auch hier möglich, jeden beliebigen Latex als Kernmaterial zu verwenden, sofern er die genannten Forderungen, wie z.B. Formstabilität und hohe Dichte erfüllt. Für die Herstellung des feinerteiligen Kern materials sind als Verfahren ein- oder mehrstufige Batch-, Monomer- oder Emulsionszulauf oder kontinuierliche Fahrweise geeignet. Als Initiatoren können die im vorangegangenen Abschnitt für die Herstellung des großteiligen Kern-Latex genannten wasser- bzw. öllöslichen Starter verwendet werden. Die Polymerisation kann, wie dort angegeben, rein thermisch oder mit Hilfe eines Redox-Systems erfolgen. Als Emulgatoren eignen sich im Prinzip alle anionischen, kationischen, nichtionischen oder amphoteren Tenside einzeln oder in Kombination, jedoch sind anionische und/oder nichtionische Emulgatoren bevorzugt. Eine besonders vorteilhafte Ausführungsform für eine Herstellung feinerteiligen Kern-Latices besteht darin, daß man eine zweckmäßigerweise puffer- (etwa pH 7) und emulgatorhaltige Lösung auf die gewünschte Polymerisationstemperatur aufheizt, einen wasserlöslichen Initiator zu gewissen Anteilen zugibt und dann über eine Zeit von 0,5 bis 6 Stunden eine Monomeremulsion (inklusive Vernetzer) zutropft. Feinerteilige Kerne von z.B. ca. 0,1 bis 0,5 µm Durchmesser können dann Anwendung finden, wenn sich die Dichte des Kern-Polymerisats genügend stark von der Dichte des Trägermediums bzw. der kontinuierlichen Phase unterscheidet.

Die Polymerisation der Schale auf dem Latex-Kern kann unmittelbar anschließend an die Polymerisation des Kernmaterials erfolgen. Die Verfahrensweise gleicht im Prinzip derjenigen, die weiter oben für den Saatlatex beschrieben worden ist. Das Monomergemisch der Schalenzusammensetzung, bei dem sich zweckmäßigerweise die Monomeren vom ZRX-Typ befinden, wird als solches oder als Emulsion in Wasser oder Pufferlösung über einen Zeitraum von 0,5 bis 4 Stunden dem Kern-Latex zugegeben, wobei wieder darauf zu achten ist, daß die Gesamtemulgatorkonzentration so niedrig bleibt, daß Teilchenneubildung vermieden wird. Gegebenenfalls kann es erforderlich sein, zwei verschiedene Monomerzuläufe gleichzeitig zu dosieren, wobei der eine u.U. Wasser enthält. Dies ist immer dann erforderlich, wenn die Monomeren sich nicht ineinander lösen, bzw. ein Teil der Monomeren nur in Wasser, der andere Teil sich aber im Wasser nicht löst.

Das Schalenmonomer polymerisiert unter diesen Voraussetzungen auf die vorhandenen Polymerisatkerne auf. Es kann sich als zweckmäßig erweisen, vor dem Zulauf der Schalenmonomere Initiator oder Pufferlösung nachzufüttern, insbesondere dann, wenn die Polymerisation des Latex-Kerns nicht in Pufferlösung erfolgt ist und wenn die Schalenmonomeren als Monomerenzulauf zugegeben werden.

Die Zugabe eines Puffers ist vor allem dann außerordentlich wichtig, wenn es sich bei den funktionellen Monomeren $Z'-(R)_n-X$ um hochreaktive Verbindungen handelt. So wird man die Puffergemische natürlich so abstimmen, daß eine Zerstörung dieser reaktiven Gruppen (z.B. durch Hydrolyse) im Verlauf der Synthese des Latex-Teilchens möglichst gering gehalten wird.

Die Polymerisationsbedingungen sind außer einer limitierten Emulgatorkonzentration ähnlich denen, die für die Herstellung des Kerns beschrieben sind. Einfach- oder Mehrfach-Batch sind als Verfahren möglich, jedoch wird ein Monomer- bzw. Emulsionszulauf bevorzugt. Die Polymerisation kann thermisch im Bereich von ca. 50 -

100° C oder sie kann mit Hilfe eines Redox-Initiatorsystems auch bei niedriger Temperatur erfolgen. Als Polymerisationsinitiatoren kommen bevorzugt die in der Emulsionspolymerisation üblichen wasserlöslichen Starter in Frage. Es sind prinzipiell jedoch auch öllösliche Initiatoren einsetzbar, soweit deren Zerfallstemperatur im angegebenen Temperaturbereich liegt.

Ein zweckmäßiges Verhältnis der Schalendichte zur Größe des Kerns liegt beispielsweise dann vor, wenn sich das Gewicht des Kernmaterials zu dem des Schalenmaterials wie 1: 3 bis 5: 1 verhält, jedoch auch extremere Kern-Schalen-Verhältnisse sind prinzipiell möglich. (10:1). Es versteht sich, daß der Schalenanteil im allgemeinen um so größer gewählt werden sollte je kleiner der Latex-Kern ist.

Die Latices fallen in Form von relativ niedrig-viskosen wäßrigen Dispersionen an. Der polymergehalt kann - als Anhalt - dabei z.B. im Bereich von 15 - 30 Gew.-% liegen. Prinzipiell sind jedoch Feststoffgehalte von wenigen Gewichtsprozenten bis etwa 70 Gew.-% möglich.

Anwendung der Kern-Schale-Latices zur Herstellung von diagnostischen Reagentien.

Die erfindungsgemäßen neuen Reagentien können durch Umsetzung der neuen Kern-Schale-Latices mit den biologisch wirksamen Substanzen bzw. Strukturen hergestellt werden. Die biologisch wirksamen Substanzen bzw. Strukturen können z.B. "immunologisch aktives" Material darstellen. Als "immunologisch aktives" Material können z.B. Komponenten physiologischer Flüssigkeiten, Zell- und Gewebeextrakte infrage kommen, vorausgesetzt, es steht ein immunologisches Gegenreagenz zur Verfügung bzw.dieses läßt sich erzeugen.

Als repräsentative Vertreter von immunologisch aktiven Materialien seien z.B. Aminosäuren, Peptide, Proteine, Enzyme, Lipoproteine, Glycoproteine, Lipoide, Nukleinsäuren, Polysaccharide, primäre Amine, Alkaloide, Hormone, Vitamine, Sterine und Steroide genannt.

Als immunologisch aktive Strukturen seien z.B. Mikroorganismen, wie gram-positive und gram-negative Bakterien, Spirochäten, Mycoplasma, Mycobacterien, Vibrionen, Actinomyceten, Protozoen, wie intestinale Protozoen, Amöben, Flagellaten, Sporozoen, intestinale Nematoden und Gewebenematoden (Würmer), Trematoden (Schistosomen, Egel), Cestoden, Toxoplasma, sowie Pilze, wie Sporotrichum, Cryptocoecus, Blastomyces, Histoplasma, Coccidioides, Candida, Viren und Rickettsien, wie Hunde-Hepatitis, Shope-Papillcne, Influenza A + B, Hühnerpest, Herpes-Simplex, Adenoviren, Polyane, Rous-Sarkom, Impfpocken, Poliovirus, Masern, Hundestaupe, Leukämie, Mumps, Newcastle-Krankheit, Sendai, ECHO, Maul- und Klauenseuche, Psittacosis, Rabies, Extromelia, Baumviren, weiter

Gewebe-Antigene, Hormone, wie Hypophysen-Hormon, Insulin, Glucagon, Thyroid-Hormcn, Choriongonadotropin, choriones Wachstumshormon-Prolactin, Human-Placenta-Lactogen, Enzyme, wie

Pancreas-Chymotrypsinogen, Procarboxypeptidase, Glucose-Oxidase, Lactatdehydrogenase, Uricase, Aminosäure-Oxidase, Urease, Asparaginase, Proteasen, Blutzellen-Antigene, Blutgruppensubstanzen und andere Isoantigene, wie Blutplättchen, Leucozyten,

Plasma-Proteine

Milch-Proteine

Speichel-Proteine

Urin-Proteine

Antikörper, einschließlich Auto-Antikörper genannt.

Anwendung der Kern-Schale-Latices zur Immobilisierung von Ehzymen.

Zur Umsetzung der erfindungsgemäßen Latices mit Ehzymen kann man in einfacher Weise das Ehzym in wäßrigem Milieu, vorzugsweise bei annähernd physiologischen Bedingungen, beispielsweise in einem geeigneten auf den Typ des Enzyms abgestimmten Puffer, mit einer adäquaten Menge des Latex inkubieren, vorzugsweise nicht wesentlich über Raumtemperatur und unter mäßigem Rühren. Wenn als funktionelle die Epoxy-Gruppe verwendet wird, kann z.B. ohne Einschränkung im pH-Bereich 7 - 9 gearbeitet werden.

Im allgemeinen ist für die Umsetzung ein Zeitraum von einem bis mehreren Tagen, beispielsweise 3 Tage angemessen. Das nicht kovalent gebundene Ehzym kann durch mehrmaliges Zentrifugieren (etwa bei 5000 U/min) und Redispergieren in Pufferlösung abgetrennt werden.

Die Bestimmung der Aktivität kann in Anlehnung an die bekannten enzymspezifischen Bestimmungsmethcden erfolgen. Ein besonderer Vorteil der vorliegenden Erfindung besteht darin, daß sich auch die beladenen Latices redispergieren und - beispielsweise in Form eines gefriergetrockneten Pulvers - gegebenenfalls über einen längeren Zeitraum aufbewahren lassen. Der limitierende Faktor ist allenfalls die Stabilität des fixierten biologischen Materials.

Die erfindungsgemäßen Latices können auch als Träger anderer - z.B. industriell verwertbarer Enzyme - in geeigneter Form zur Anwendung kommen. Genannt seien z.B. Acylasen, die Penicillinase, Glucose-Isomerase, Peroxidasen u.a.

Unter verschiedenen Aspekten, z.B. zur Verfolgung der Immun-Agglutination, kann es - wie bereits erwähntvorteilhaft sein, die Latices mit einem Marker, beispielsweise einem Fluoreszenz-Farbstoff, zu versehen.

Die Polymerlatices der vorliegenden Erfindung eignen sich zur Immobilisierung von Microorganismen allgemein, wobei die Umsetzungsbedingungen ähnlich sind wie bei Immobilisierung von Proteinen. Gegenüber dem Stand der Technik bietet das vorliegende Verfahren eine bessere Zugänglichkeit des Immobilisierten Microorganismus gegenüber Substratmokekülen. Hervorzuheben ist die geringe Cytotoxizität, die dem vorliegenden Immobilisierungsverfahren eigen ist.

Die oben stehenden Gesichtspunkte gelten auch für die Immobilisierung von Viren und von eukariotischen Zellen. Die polyfunktionelle Natur der Polymerlatices erlaubt auch generell ihre Verwendung zur Vernetzung von biologisch wirksamen Substanzen. Unter diesem Aspekt kommt besonders den Latexteilchen mit kleinem

Durchmesser (Richtwert ca. 500 Å) besondere Bedeutung zu.

Auch in der präparativen organischen Synthese können die Polymerlatices der Erfindung mit Vorteil angewendet werden, wobei nicht notwendigerweise in wäßrigem Milieu gearbeitet zu werden braucht, sondern auch organische Reaktionsmedien (mit) verwendet werden können.

Es können z.B. in dieser Weise Schutzgruppen eingeführt werden. Ein besonders interessanter Aspekt liegt in der Verwendung im Sinne einer Peptidsynthese nach Merrifield. (Vgl. Merrifield, Adv. Enzymol. 32 (1969) 221-296).

**1. Herstellung des Latex 1**

(Beispiel für einen grobteiligen Latex)

a) Synthese der Stammdispersion

In einem Polymerisationsgefäß, ausgestattet mit Rückflußkühler, Rührer und Thermometer, werden 1600 g Wasser vorgelegt und auf 80° C erwärmt. Nach Zugabe einer Monomerenmischung, bestehend aus

3 g Isobutylmethacrylat
3 g Methylmethacrylat
0,3 g Äthylenglykoldimethacrylat

setzt man 4 g Ammoniumpersulfat, gelöst in 36 g Wasser, zu.

Darauf tropft man ebenfalls bei 80° C eine Mischung aus

200 g Isobutylmethacrylat
200 g Methylmethacrylat
20 g Äthylenglykoldimethacrylat

innerhalb von 2 Std. zu. Nach Ende der Monomerenzugabe wird noch 1 Std. bei 80° C belassen. Man erhält eine koagulatfreie, gut filtrierbare, niedrig-viskose, ca. 20 %ige Dispersion.

b) Synthese der oxirangruppenhaltigen Dispersion

In einem Polymerisationsgefäß, ausgestattet mit Rückflußkühler, Rührer und Thermometer, werden 350 ml Wasser vorgelegt. Dazu gibt man 10 ml einer Phosphatpufferlösung pH 7 (Titrisol, Merck) und 80 g der Stammdispersion. Nach Erhitzen auf 80° C setzt man 0,4 g Na-Salz der 4,4'-Azobis-(4-cyanovaleriansäure) Natriumsalz in 4 ml Wasser zu.

Danach dosiert man eine Emulsion, bestehend aus

1000 g Wasser
1 g Natriumlaurylsulfat
2 g Na-Salz der 4,4'-Azobis-(cyänovaleriansäure)
150 g Methylmethacrylat
150 g Isobutylmethacrylat
15 g Äthylenglykoldimethacrylat

innerhalb von 3 Std. bei 80° C zu.

Im Anschluß daran werden innerhalb von 60 Min. nebeneinander eine Lösung von 20 g Methacrylsäureamid und 0,6 g Na-Salz der 4,4'-Azobis(4-cyanovaleriansäure) in 300 g Wasser sowie eine Monomerenmischung, bestehend aus 35 g Methylmethacrylat, 40 g Glycidylmethacrylat sowie 4 g Äthylenglykoldimethacrylat zugegeben. Danach wird noch weitere 60 Min. bei 80° C gerührt. Man erhält eine koagulatfreie, niedrigviskose Dispersion mit einem Feststoffgehalt von ca. 20 % Teilchengröße ca. 2 µm.

**2. Herstellung des Latex 2**

(Beispiel für einen grobteiligen Latex)

a) Synthese der Stammdispersion

In einem Polymerisationsgefäß gemäß Beispiel werden 1600 g Wässer vorgelegt und auf 80° C erwärmt. Nach Zugabe einer Monomerenmischung, bestehend aus

6,24 g Styrol und
0,06 g Allylmethacrylat

setzt man 4 g Ammoniumpersulfat, gelöst in 36 g Wasser, zu. Dazu tropft man ebenfalls bei 80° C eine Mischung aus

415 g Styrol und
5 g Allylmethacrylat

innerhalb von 2 Std. zu.

Nach Ende der Monomerenzugabe wird noch 2 Std. bei 80° C belassen. Man erhält eine koagulatfreie, grob filtrierbare viskose, ca. 20 %ige Dispersion.

b) Synthese der oxirangruppenhaltige Dispersion

Man verfährt wie in Beispiel 1, erhitzt jedoch auf 85° C und setzt 1,0 g Na-Salz der 4,4'-Azobis (4-cyanovaleriansäure) Natriumsalz in 10 ml Wasser zu.

Dazu dosiert man eine innerhalb von 3 Std. bei 85° C Emulsion, bestehend aus:

1000 g Wasser
1 g Natriumlaurylsulfat
4 g Na-Salz der 4,4'-Azobis-(cyanovaleriansäure)
312 g Styrol
4 g Allylmethacrylat.

Im Anschluß daran werden innerhalb von 90 Min. nebeneinander eine Lösung von 20 g Methacrylsäureamid und 0,6 g Na-Salz der 4,4'-Azobis-(4-cyanovaleriansäure) in 300 g Wasser sowie eine Monomerenmischung, bestehend aus 35 g Methylmethacrylat, 40 g Glycidylmethacrylat sowie 4 g Äthylenglykoldimethacrylat

zugegeben. Danach wird noch 60 Min. bei 80° C gerührt.

Man erhält eine koagulatfreie, gut filtrierbare niedrig-viskose Dispersion mit einem Feststoffgehalt von ca. 20 %. Teilchengröße: ca. 2 µm.

**3. Herstellung des Latex 3**

(Beispiel für feinteiligen Latex)

In einem Polymerisationsgefäß mit der oben beschriebenen Ausstattung werden 5 ml phosphatpufferlösung (pH 7, Titrisol, Merck) 0,03 g Natriumlaurylsulfat und 0,2 g Na-Salz der 4,4'-Azobis-(4-cyanovaleriansäure) in 100 ml Wasser gelöst. Man erhitzt auf 80° C umd tropft innerhalb von 3 Std. eine Emulsion zu, bestehend aus 0,1 g Natriumlaurylsulfat, 0,5 g Na-Salz der 4,4'-Azobis(4-cyanovaleriansäure), 80 g Methylmethacrylat, 15 g (2-Äthyl-[2,4,6-Tribromphenoxy]-äthyl)-methacrylat, 5 g Äthylenglykoldimethacrylat und 200 g Wasser. Anschließend gibt man innerhalb von 90 Min. gleichzeitig in den Reaktionsansatz eine Lösung von 5 g Methacrylsäureamid in 75 g Wasser und eine Monomerenmischung, bestehend aus

10 g Glycidylmethacrylat
1 g Äthylenglykoldimethacrylat
9 g Methylmethacrylat.

Danach hält man noch etwa 60 Min. bei 80° C.

Es resultiert eine ca. 25 %ige, niedrig-viskose Dispersion.

Teilchengröße: 0,3 µm.

Gehalt an Oxirangruppen: 31 % des eingesetzten Glycidylmethacrylates (Titration mit Natriumthiosulfat).

**4. Herstellung des Latex 4**

**4a Herstellung der Kern-Dispersion**

In einem Polymerisationsgefäß mit der im Beispiel 1 beschriebenen Ausstattung werden
0,3 g Natriumtetradecylsulfonat
0,6 g Ammoniumpersulfat und
500 g destilliertes Wasser vorgelegt und auf
80° C erwärmt.

In diese Vorlage tropft man bei 80° C im Verlauf von 6 Std. eine Emulsion bestehend aus:
500 g p-Bromstyrol
300 g Fumarsäurediäthylester
4 g Natriumtetradecylsulfonat
4 g Ammoniumpersulfat und
710 g destilliertes Wasser.

Nach Zulaufende wird weitere 2 Std. bei 80° C gerührt, danach auf Raumtemperatur abgekühlt und filtriert. Die Dispersion ist niedrig-viskos, sie hat einen Feststoffgehalt von ca. 40 %.

**4b Herstellung der Kern-Schale-Dispersion**

500 g der 40 %igen Dispersion 4a werden mit Phosphatpuffer auf pH 7,0 eingestellt und verdünnt mit einer Lösung, bestehend aus 1 g des Natriumsalzes der 4,4'-Azobis-(cyanovaleriansäure) und 0,5 g Natriumtetradecylsulfonat in 1000 ml destilliertem Wasser auf ein Volumen von insgesamt 1000 ml.

(= 20 %ige Dispersion 4a vom pH 7,0).

Man erhitzt diese Vorlage in einem Polymerisationsgefäß auf 80° C, hält 15 Min. bei dieser Temperatur und tropft dann nebeneinander bei 80° C die folgenden 2 Lösungen ein:

**Lösung A:** 20 g 2-Bromäthylmethacrylat
2,5 g Glykoldimethacrylat
17 5 g N-t.Butylmethacrylamid
10 g Methylmethacrylat

**Lösung B:** 1 g Natriumsalz der 4,4'-Azobis
(cyanovaleriansäure) in
50 g destilliertem Wasser

Zutropfdauer: ca. 2 Std. Die Dosiergeschwindigkeit sollte in beiden Zuläufen möglichst gleich groß sein. Nach Beendigung des Zulaufs wird noch 1 Std. bei 80° C belassen. Danach wird abgekühlt und filtriert. Es resultiert eine feinteilige, niedrig-viskose Dispersion mit einem Feststoffgehalt von ca. 23 %.

**4c Herstellung der Kern-Schale-Dispersion**

Man verfährt wie im Beispiel 4b (Verdünnung der Dispersion 4a, Neutralisation etc.), dosiert jedoch die folgenden Lösungen

**Lösung A:** 10 g Vinylacetat
30 g Chloressigsäurevinylester
2,5 g Methylenbisacrylamid
7,5 g Acrylamid

**Lösung B:** 2 g Natriumsalz der 4,4'-Azobis
(cyanovaleriansäure) in
50 g destilliertem Wasser

Zutropfdauer: ca. 3 Std.,nach Beendigung des Zulaufs wird noch 2 Std. bei 80° C belassen. Nach Abkühlen und Filtrieren resultiert eine feinteilige, niedrig-viskose Dispersion.

**5. Synthese des Latex 5**

**Stufe I**

In einem Polymerisationsgefäß gemäß Beispiel 1 werden die folgenden Komponenten vorgelegt
1550 g destilliertes Wasser
0,8 g Natriumlaurylsulfat
3,2 g Methylmethacrylat
3,2 g Isobutylmethacrylat und unter Rühren auf 80°C erwärmt. Im Anschluß daran setzt man eine Lösung von 4 g Ammoniumpersulfat in 40 ml Wasser zu. Anschließend dosiert man bei 80°C eine Monomermischung, bestehend aus:
190 g Methylmethacrylat
190 g Isobutylmethacrylat
20 g Glykolbismethacrylat
Dauer des Monomerenzulaufs: 2 Std.
Nach Zulaufende wird weitere 2 Std. bei 80°C belassen. Nach dem Abkühlen resultiert eine gut filtrierbare, koagulatfreie Dispersion: Feststoffgehalt: 19 %, pH 2,2. Viskosität 4 mPa.sec.

**Stufe II**

In einem Polymerisationsgefäß gemäß Beispiel 1 werden 160 g der Dispersion Stufe I vorgelegt, dazu gibt man
10 g Phosphatpuffer pH 7 (Titrisol, Merck)
0,4 g Natriumsalz der 4,4'-Azobis-(cyano-valeriansäure)
310 g destilliertes Wasser
Man erwärmt diese Vorlage auf 80°C und dosiert innerhalb von 3 Std. die folgende Emulsion:
143 g Methylmethacrylat
143 g Isobutylmethacrylat
15 g Äthylenglykolbismethacrylat
1 g Natriumlaurylsulfat
1,8 g Natriumlaurylsulfat
970 g destilliertes Wasser.
Sofort im Anschluß daran dosiert man nebeneinander die beiden folgenden Mischungen (Dauer: 1 Stunde).
**Mischung A:** 44 g Methylmethacrylat
4 g Äthylenglykolbismethacrylat
42 g Glycidylmethacrylat
**Mischung B:** 0,6 g Natriumsalz der 4,4'-Azobis-(cyanovaleriansäure)
10 g Methacrylamid
320 g destilliertes Wasser.
Nach Zulaufende wird eine weitere Stunde bei 80°C belassen. Nach dem Abkühlen resultiert eine koagulatfreie Dispersion: Feststoffgehalt: ca. 19 %. Teilchengröße ca. 0,4 µm.

**6. Reinigung des Latex gemäß Beispiel 1**

(Entfernung der synthesebedingten Hilfsstoffe, Emulgatoren, Initiatoren etc.)

10 ml der Dispersion 1 werden 15 Min. bei 5000 U/min zentrifugiert. Das überstehende Serum wird abgegossen, anschließend werden die Teilchen in 1 n NaCl redispergiert (1 g Polymerisatfeststoff in ca. 50 ml 1 n NaCl). Danach wird 10 Min. bei 5000 U/min. zentrifugiert umd dekantiert. Das Redispergieren in 1 n NaCl und Zentrifugieren wird noch zweimal wiederholt.

In Anschluß daran werden die Teilchen in 0,05 m Phosphatpuffer pH 7,5 redispergiert (1 g Polymerisatfeststoff in 50 ml 0,05 m Phosphatpuffer pH 7,5). Es wird 10 Min. bei 5000 U/min zentrifugiert umd der Überstand abgegossen.

Dieser Vorgang wird einmal wiederholt. Die Lagerung des so gewonnenen Latex erfolgt im Kühlschrank bei +5°C.

**7. Reinigung des Latex gemäß Beispiel 3**

Man verfährt wie im Beispiel 6, erhöht jedoch die Zentrifugationsdauer auf jeweils 30 Min. (5000 U/min).

**8. Umsetzung zur Immobilisierung von Trypsin**

15 ml der Dispersion gemäß Beispiel 1 (≙ 3 g Polymerisatfestsubstanz) werden mit 300 mg Trypsin (gelöst in 6 ml 1 m Phosphatpuffer pH 7,5) versetzt, anschließend wird 72 Std. bei 23°C gerührt.

Nicht kovalent gebundenes Enzym wird danach durch dreimaliges Zentrifugieren und Redispergieren im 0,05 m Phosphatpuffer entfernt (Durchführung gemäß Eeispiel 6).

**9. Messung der Aktivität des immobilisierten Enzyms**

**a) Hydrolyse von N.Benzoyl-Arginin-Äthylester (BAEE) bei 37°C und pH 7,5 (pH-Stat)**

1 g der Trockensubstanz des durch Zentrifugation gereinigten Latex gemäß Beispiel 8 (eingesetzt als ca. 2 g Feuchtsubstanz mit ca. 1 g Wasser) werden in 20 ml einer 2 %igen BAEE-Lösung dispergiert.

| Verwendung | Aktivität [U/g] *) |
|---|---|
| 1. Verwendung | 14,2 |
| 2. Verwendung | 12,1 |
| 3. Verwendung | 11,8 |
| 4. Verwendung | 11,8 |

*) Aktivitäten jewils bezogen auf g Trägermaterial,

ein U entspricht 1 µmol/min, gemessen anhand der Anfangsgeschwindigkeit.

**b) Hydrolyse von Casein (37°C, pH 8,0)**

1 g der Trockensubstanz des durch Zentrifugieren gereinigten Latex gemäß Beispiel 8 (eingesetzt als ca. 2 g Beuchtsubstanz mit ca. 1 g Wasser) werden in 20 ml einer 4 %igen Casein-Lösung dispergiert.

| Verwendung | Aktivität [U/g Trägermaterial] |
|---|---|
| 1. Verwendung | 3,2 |
| 2. Verwendung | 2,6 |
| 3. Verwendung | 2,6 |
| 4. Verwendung | 2,6 |

**10. Gefriertrocknung eines reaktiven Latex**

15 ml der Dispersion gemäß Beispiel 1 werden - wie im Beispiel 6 beschrieben - gereinigt. Dabei resultiert ein Polymerisat mit ca. 50 % Restfeuchte.

Dieser zentrifugierte Latex wird gefriergetrocknet und anschließend bei -20°C 6 Monate gelagert.

**Redispergieren des gefriergetrockneten Latex:**

Das Redispergieren erfolgt mit 0,05 molarem Phosphatpuffer pH 7,5. Dabei muß ca. 5 Min. kräftig gerührt werden. Alternativ kann auch die im Puffer suspendierte Probe kurz mit Ultraschall behandelt werden.

Anschließend erfolgt die Umsetzung mit dem Enzym wie im Beispiel 8 beschrieben (10 % Trypsin bezogen auf den eingesetzten Latex).

Aktivität des gefriergetrockneten, 6 Monate bei -20°C gelagerten, redispergierten und mit 10 % Trypsin umgesetzten Latex:

| Verwendung | Aktivität [U/g] *) (Substrat: BAEE) | Aktivität [U/g] *) (Substrat: Casein) |
|---|---|---|
| 1. Verwendung | 13,3 | 2,9 |
| 2. Verwendung | 10,6 | 2,2 |
| 3. Verwendung | 10,6 | 2,2 |

*) bezogen auf g Trägermaterial

**11. Gefriertrocknung eines Latex mit immobilisiertem Trypsin**

1 g des mit Trypsin umgesetzten Latex (Beispiel 8) wird gefriergetrocknet und danach 6 Monate bei -20°C gelagert. Das Redispergieren erfolgt - wie im Beispiel 10 beschrieben mit 0,05 m Phosphatpuffer.

Aktivität gegenüber Casein (1 g Festsubstanz des redispergierten Latex in 20 ml 4 %ige Caseinlösung) 37°C, pH 8,0

| Verwendung | Aktivität [U/g Trägermaterial] |
|---|---|
| 1. Verwendung | 3,6 |
| 2. Verwendung | 2,4 |
| 3. Verwendung | 2,4 |

**12. Immobilisierung von Trypsin**

Man verfährt wie im Beispiel 8, verwendet zur Immobilisierung des Trypsins jedoch 15 ml der Dispersion gemäß Beispiel 3 (Zentrifugation 30 Min. bei 5000 U/min).

Aktivitäten gegenüber Casein als Substrat (pH 8,0, 37°C)

1. Verwendung 5,5 U/g Trägermaterial
2. Verwendung 4,2 U/g Trägermaterial
3. Verwendung 4,0 U/g Trägermaterial

**13. Synthese eines fluoreszenzmarkierten Latex**

In einem Polymerisationsgefäß gemäß Beispiel 1 werden 40 g der Stammdispersion Ia vorgelegt, dazu gibt man 5 ml Phosphatpuffer pH 7 (Titrisol, Merck), 0,2 g Natriumsalz der 4,4'-Azobis-(cyanovaleriansäure) und 180 g destilliertes Wasser.

Nach Erwärmen der Vorlage auf 80°C dosiert man - ebenfalls bei 80°C - innerhalb von 3 Std. eine Emulsion, bestehend aus:

127 g Methylmethacrylat
15 g Isobutylmethacrylat
7,5 g Äthylenglykolbismethacrylat
0,6 g Flurol-Grün-Gold
1,0 g Natriumsalz der 4,4'-Azobis-(cyano-valeriansäure)
0,5 g Natriumlaurylsulfat
450 g destilliertes Wasser

Nach Beendigung dieses Zulaufs (= Latex-Kern) dosiert man bei 80°C in 1 Std. nebeneinander die beiden folgenden Mischungen zu:

**Mischung A:** 24 g Methylmethacrylat
2 g Äthylenglykolbismethacrylat
21 g Glycidylmethacrylat

**Mischung B:** 3 g Methacrylamid
0,3 g Natriumsalz der 4,4'-Azobis-(cyanovaleriansäure)
155 g destilliertes Wasser

Nach Beendigung des Zulaufs wird noch 60 Min. bei 80°C belassen, danach wird abgekühlt.

Es resultiert eine gut filtrierbare, koagulatfreie Dispersion mit einem Feststoffgehalt von 19 %, pH: 7,7, Viskosität: 10 mPa.sec.

Teilchengröße: 2 µm

Die Fluoreszenz bei UV Anregung ist sowohl makroskopisch als auch im Fluoreszenzmikroskop deutlich sichtbar.

**14. Immobilisierung von Antialbumin**

10 ml der Dispersion gemäß Beispiel 5 werden mit 0,05 m Phosphatpuffer pH 7,5 auf 100 ml verdünnt. (Dem Phosphatpuffer wird zweckmäßigerweise 0,05 % Natriumazid zugesetzt). Es resultiert eine Dispersion mit ca. 2 % Polymerisatfeststoff.

Das Antiserum Cat.No. 61-015,6389 (Ziege) wird mit Puffer auf die folgenden Konzentrationen verdünnt.

a) 1000 µg AK/ml
b) 200 µg AK/ml
c) 40 µg AK/ml
d) 8 µg AK/ml
e) 0 µg AK/ml

Die Bindung des Antialbumins an die Latex-Teilchen erfolgt durch Umsetzung von jeweils 1 ml der 2 %igen Dispersion mit 1 ml der Verdünnungsreihe a) bis e). Man rührt 5 Tage bei Raumtemperatur und reinigt die Latex-Teilchen wie im Beispiel 6 beschrieben durch Zentrifugation.

**Patentansprüche**

1. Polymerlatices mit Kern-Schale-Aufbau zur Fixierung biologisch wirksamer Substanzen,
dadurch gekennzeichnet,
daß die Polymerlatices redispergierbar sind, wobei das Polymermaterial der Schale
I) zu 0,1 bis 20 Gew.-% aus einem radikalisch polymerisierbaren Vernetzer
II) zu 4,9 bis 99,9 Gew.-% aus radikalisch polymerisierbaren funktionellen Monomeren der Formel
$Z'-(R)_n-X$
wobei Z' für die radikalisch polymerisierbare Einheit
R für einen Abstandhalter
X fur eine reaktive, nucleophil angreifbare Gruppe und
n für 0 oder 1 steht
zusammen mit radikalisch polymerisierbaren, hydrophilen Monomeren B mit der Maßgabe, daß der Anteil der funktionellen Monomeren am Gesamtpolymerisat der Schale wenigstens 0,1 Gew.-% beträgt, und
III) zu 0 bis 95 Gew.-% aus nicht oder nur begrenzt wasserlöslichen, radikalisch polymerisierbaren Monomeren A aus der Gruppe der Methacrylsäure- und Acrylsäureester sowie aus der Gruppe der Vinylester von Carbonsäuren aufgebaut ist, wobei die Monomerenzusammensetzung der Schale so gewählt ist, daß
IV) der $T_{\lambda\ max}$-Wert der Schale im wasserfreien Zustand in Bereich von 20°C bis 250°C liegt,
und daß das Polymermaterial des Kerns durch Emulsionspolymerisation von radikalisch polymerisierbaren Monomeren erzeugt worden ist.

2. Polymerlatices mit Kern-Schale-Aufbau gemäß Anspruch 1, dadurch gekennzeichnet, daß das Polymermaterial der Schale keine aromatischen Struktureinheiten besitzt.

3. Polymerlatices mit Kern-Schale-Aufbau gemäß den Ansprüchen 1 und 2, dadurch gekennzeichnet, daß das Polymermaterial der Schale elektroneutral ist.

4. Polymerlatices mit Kern-Schale-Aufbau gemäß den Ansprüchen 1 bis 3, dadurch gekennzeichnet, daß die Monomeren der Typen A und B jeweils zu mindestens 50 Gew.-% bezogen auf das Gesamtgewicht der Monomeren A oder B, aus Derivaten der Acryl- und/oder der Methacrylsäure bestehen.

5. Polymerlatices mit Kern-Schale-Aufbau gemäß den Ansprüchen 1 bis 4, dadurch gekennzeichnet, daß der Kern aus einem an sich weichen, aber stark vernetzten Polymermaterial aufgebaut ist.

6. Polymerlatices mit Kern-Schale-Aufbau gemäß den Ansprüchen 1 bis 4, dadurch gekennzeichnet, daß der Kern aus einem an sich harten Polymermaterial aufgebaut ist.

7. Polymerlatices mit Kern-Schale-Aufbau gemäß den Ansprüchen 1 bis 6, dadurch gekennzeichnet, daß der Kern Träger einer auf physikalischem Wege feststellbaren Markierung ist.

8. Polymerlatices mit Kern-Schale-Aufbau gemäß den Ansprüchen 1 bis 7, dadurch gekennzeichnet, daß der Kern einen oder mehrere Farbstoffe enthält.

9. Polymerlatices mit Kern-Schale-Aufbau gemäß dem Anspruch 8, dadurch gekennzeichnet, daß der Kern einen oder mehrere Fluoreszenzfarbstoffe enthält.

10. Polymerlatices mit Kern-Schale-Aufbau gemäß den Ansprüchen 1 bis 9, dadurch gekennzeichnet, daß der Kern eine solche Dichte besitzt, daß dem gesamten Polymerlatex eine von dem anzuwendenden wäßrigen Medium abweichende Dichte verliehen wird.

11. Polymerlatices mit Kern-Schale-Aufbau gemäß Anspruch 10, dadurch gekennzeichnet, daß der Kern ganz oder teilweise aus solchen Monomeren aufgebaut ist, die dem gesamten Polymerlatex eine höhere Dichte als die des wäßrigen Mediums verleihen.

12. Polymerlatices mit Kern-Schale-Aufbau gemäß den Ansprüchen 1 bis 11, dadurch gekennzeichnet, daß der mittlere Teilchendurchmesser zwischen 0,05 und 5 µm liegt.

13. Polymerlatices mit Kern-Schale-Aufbau gemäß Anspruch 12, dadurch gekennzeichnet, daß der mittlere Teilchendurchmesser 0,5 bis $>$ 2 µm beträgt.

14. Polymerlatices mit Kern-Schale-Aufbau gemäß den Ansprüchen 1 bis 13, dadurch gekennzeichnet, daß das Gewicht des Polymermaterials des Kerns zu dem Gewicht des Polymermaterials der Schale im Verhältnis 1: 3 bis 10 : 1 steht.

15. Polymerlatices mit Kern-Schale-Aufbau gemäß den Ansprüchen 1 bis 14, dadurch gekennzeichnet, daß der Gewichtsanteil der Schale umso größer gewählt wird, je kleiner der Latex-Kern ist.

16. Wäßrige Dispersionen der Polymerlatices gemäß den Ansprüchen 1 bis 15.

17. Wäßrige Dispersionen der Polymerlatices gemäß Anspruch 16, dadurch gekennzeichnet, daß sie einen Polymergehalt von 15 bis 30 Gew.-%, bezogen auf die Gesamtdispersion, besitzen.

18. Verwendung der Polymerlatices gemäß den Ansprüchen 1 bis 17, zur Herstellung von diagnostischen Reagentien.

19. Verwendung der Polymerlatices gemäß Anspruch 18, zur Herstellung von Diagnostika zum Nachweis von Antigen-Antikörperreaktionen.

20. Verwendung der Polymerlatices gemäß Anspruch 1, zur Immobilisierung von Antikörpern.

21. Verwendung der Polymerlatices gemäß den Ansprüchen 1 bis 17, zur Immobilisierung von Microorganismen.

22. Verwendung der Polymerlatices gemäß den Ansprüchen 1 bis 17, zur Immobilisierung von Viren.

23. Verwendung der Polymerlatices gemäß den Ansprüchen 1 bis 17, zur Immobilisierung von einem oder mehreren Enzymen.

24. Verwendung der Polymerlatices gemäß den Ansprüchen 1 bis 17, zum Vernetzen von Bio(makro)molekülen.

## Claims

1. Polymer lattices with a core-shell structure for fixing biologically active substances, characterised in that the polymer lattices are redispersible, the polymer material of the shell being made up of

I) 0.1 to 20% by weight of a radically polymerisable cross-linking agent

II) 4.9 to 99.9% by weight of radically polymerisable functional monomers of formula

$Z'-(R)_n-X$

wherein

Z' represents the radically polymerisable unit

R represents a spacer

X represents a reactive, nucleophilically attackable group and

n represents 0 or 1

together with radically polymerisable hydrophilic monomers B, with the proviso that the proportion of functional monomers in the total polymer of the shell amounts to at least 0.1% by weight, and

III) 0 to 95% by weight of radically polymerisable monomers A which are not water-soluble or only water soluble to a limited extent, from the group of methacrylic acid and acrylic acid esters and from the group of vinyl esters of carboxylic acids, the monomer composition of the shell being chosen so that

IV) the $T_{\lambda max}$ value of the shell in the anhydrous state is in the range from 20°C to 250°C,

and in that the polymer material of the core has been produced by emulsion polymerisation of radically polymerisable monomers.

2. Polymer lattices with a core-shell structure as claimed in claim 1, characterised in that the polymer material of the shell has no aromatic structural units.

3. Polymer lattices with a core-shell structure as claimed in claims 1 and 2, characterised in that the polymer material of the shell is electroneutral.

4. Polymer lattices with a core-shell structure as claimed in claims 1 to 3, characterised in that the monomers of types A and B each consist of at least 50% by weight, based on the total weight of monomers A or B, of derivatives of acrylic and/or methacrylic acid.

5. Polymer lattices with a core-shell structure as claimed in claims 1 to 4, characterised in that the core is made up of a polymer material which is inherently soft but strongly cross-linked.

6. Polymer lattices with a core-shell structure as claimed in claims 1 to 4, characterised in that the core is made up of an inherently hard polymer material.

7. Polymer lattices with a core-shell structure as claimed in claims 1 to 6, characterised in that the core is the carrier of a marking which can be detected by physical methods.

8. Polymer lattices with a core-shell structure as claimed in claims 1 to 7, characterised in that the core contains one or more dyes.

9. Polymer lattices with a core-shell structure as claimed in claim 8, characterised in that the core contains one or more fluorescent dyes.

10. Polymer lattices with a core-shell structure as claimed in claims 1 to 9, characterised in that the core has a density such that a density different from that of the aqeuous medium used is imparted to the entire polymer latex.

11. Polymer lattices with a core-shell structure as claimed in claim 10, characterised in that the core is made up entirely or partially of monomers of the kind which impart, to the polymer latex as a whole, a greater density than that of the aqueous medium.

12. Polymer lattices with a core-shell structure as claimed in claims 1 to 11, characterised in that the average particle diameter is between 0.05 and 5 microns.

13. Polymer lattices with a core-shell structure as claimed in claim 12, characterised in that the average particle diameter is 0.5 to 2 microns.

14. Polymer lattices with a core-shell structure as claimed in claims 1 to 13, characterised in that the ratio of the weight of the polymer material of the core to the weight of the polymer material of the shell is from 1:3 to 10:1.

15. Polymer lattices with a core-shell structure as claimed in claims 1 to 14, characterised in that the proportion by weight represented by the shell is greater, the smaller the latex core.

16. Aqueous dispersions of the polymer lattices as claimed in claims 1 to 15.

17. Aqueous dispersions of the polymer lattices as claimed in claim 16, characterised in that they have a polymer content of from 15 to 30% by weight, based on the total dispersion.

18. Use of the polymer lattices as claimed in claims 1 to 17 for preparing diagnostic reagents.

19. Use of the polymer lattices as claimed in claim 18 for preparing diagnostic agents for detecting antigen-antibody reactions.

20. Use of the polymer lattices as claimed in claim 1 for immobilising antibodies.

21. Use of the polymer lattices as claimed in claims 1 to 17 for immobilising microorganisms.

22. Use of the polymer lattices as claimed in claims 1 to 17 for immobilising viruses.

23. Use of the polymer lattices as claimed in claims 1 to 17 for immobilising one or more enzymes.
24. Use of the polymer lattices as claimed in claims 1 to 17 for cross-linking bio(macro)molecules.

**Revendications**

1. Latex polymères à structure de noyau-capsule pour la fixation de substances biologiquement actives, caractérisés en ce que les latex polymères sont susceptibles de redispersion, la matière polymère de la capsule étant faite

I) pour 0,1 à 20 % en poids, d'un réticulant susceptible de polymérisation radicalaire,

II) pour 4,9 à 99,9 % en poids, de monomères fonctionnels susceptibles de polymérisation radicalaire de formule

$Z'-(R)_n-X$

Z' étant mis pour l'unité susceptible de polymérisation radicalaire,
R pour un distanciateur,
X pour un groupe réactif susceptible d'attaque nucléophile,
n pour 0 ou 1,

avec des monomères hydrophiles B, susceptibles de polymérisation radicalaire, avec cette condition que la part des monomères fonctionnels dans le polymère total de la capsule s'élève au moins à 0,1 % en poids, et

III) pour 0 à 95 % en poids, de monomères A susceptibles de polymérisation radicalaire, insolubles ou seulement solubles de façon limitée dans l'eau, choisis dans le groupe des esters d'acide méthacrylique et d'acide acrylique, ainsi que dans le groupe des esters vinyliques d'acides carboxyliques, la composition des monomères de la capsule étant choisie de telle manière que

IV) la valeur $T_{\lambda max}$ de la capsule à l'état anhydre se situe dans la gamme de 20° C à 250° C,

et en ce que la matière polymère du noyau est obtenue par polymérisation en émulsion de monomères susceptibles de polymérisation radicalaire.

2. Latex polymères à structure de noyau-capsule selon la revendication 1, caractérisés en ce que la matière polymère de la capsule ne comporte pas d'unités structurales aromatiques.

3. Latex polymères à structure de noyau-capsule selon la revendication 1 ou 2, caractérisés en ce que la matière polymère de la capsule est électriquement neutre.

4. Latex polymères à structure de noyau-capsule selon l'une quelconque des revendications 1 à 3, caractérisés en ce que les monomères de chacun des types A et B sont faits, pour 50 % en poids au moins par rapport au poids total des monomères A ou B, de derivés de l'acide acrylique et/ou méthacrylique.

5. Latex polymères à structure de noyau-capsule selon l'une quelconque des revendications 1 à 4, caractérisés en ce que le noyau est constitué d'une matière polymère molle en soi, mais fortement réticulée.

6. Latex polymères à structure de noyau-capsule selon l'une quelconque des revendications 1 à 4, caractérisés en ce que le noyau est constitué d'une matière polymère dure en soi.

7. Latex polymères à structure de noyau-capsule selon l'une quelconque des revendications 1 à 6, caractérisés en ce que le noyau est porteur d'un marquage décelable par voie physique.

8. Latex polymères à structure de noyau-capsule selon 1,une quelconque des revendications 1 à 7, caractérisés en ce que le noyau contient un ou plusieurs colorants.

9. Latex polymères à structure de noyau-capsule selon la revendication 8, caractérisés en ce que le noyau contient un ou plusieurs colorants fluorescents.

10. Latex polymères à structure de noyau-capsule selon l'une quelconque des revendications 1 à 9, caractérisés en ce que le noyau a une densite telle qu'il soit donné, au latex polymère total, une densité qui s'écarte de celle du milieu aqueux qui doit être utilisé.

11. Latex polymères à structure de noyau-capsule selon la revendication 10, caractérisés en ce que le noyau est fait en totalité ou partiellement de monomères qui donnent, au latex polymère total, une densité plus élevée que celle du milieu aqueux.

12. Latex polymères à structure de noyau-capsule selon l'une quelconque des revendications 1 à 11, caractérisés en ce que le diamètre moyen des particules se situe entre 0,05 et 5µm.

13. Latex polymères à structure de noyau-capsule selon la revendication 12, caractérisés en ce que le diamètre moyen des particules mesure 0,5 a plus de 2µm.

14. Latex polymères à structure de noyau-capsule selon l'une quelconque des revendications 1 à 13, caractérisés en ce que le poids de la matière polymère du noyau est, au poids de la matière polymère de la capsule, dans un rapport de 1: 3 à 10: 1.

15. Latex polymères à structure de noyau-capsule selon l'une quelconque des revendications 1 à 14, caractérisés en ce que la part de poids de la capsule est choisie d'autant plus grande que le noyau de latex est plus petit.

16. Dispersions aqueuses des latex polymères selon l'une quelconque des revendications 1 à 15.

17. Dispersions aqueuses des latex polymères selon la revendication 16, caractérisées en ce qu'elles ont une teneur en polymères de 15 à 30 % en poids par rapport à la dispersion totale.

18. Utilisation des latex polymères selon l'une quelconque des revendications 1 à 17 pour la préparation de réactifs à usage diagnostique.

19. Utilisation des latex polymères selon la revendication 18 pour la préparation de moyens de diagnostic pour

la mise en évidence de réactions antigène-anticorps.

20. Utilisation des latex polymères selon la revendication 1 pour l'immobilisation d'anticorps.

21. Utilisation des latex polymères selon l'une quelconque des revendications 1 à 17 pour l'immobilisation de microorganismes.

22. Utilisation des latex polymères selon l'une quelconque des revendications 1 à 17 pour l'immobilisation de virus.

23. Utilisation des latex polymères selon l'une quelconque des revendications 1 à 17 pour l'immobilisation d'un ou de plusieurs enzymes.

24. Utilisation des latex polymères selon l'une quelconque des revendications 1 à 17 pour la réticulation de bio(macro)molécules.